# EUROPEAN PATENT SPECIFICATION

(11) **EP 0 814 782 B1**
(45) Date of publication and mention of the grant of the patent: **27.11.2002**
(21) Application number: 96942588.3
(22) Date of filing: 20.12.1996
(51) Int. Cl.: A61K 9/20, A61K 31/47

(54) **PHARMACEUTICAL COMPOSITION STABILIZED WITH A BASIC AGENT**
PHARMAZEUTISCHE ZUSAMMENSETZUNG STABILISIERT MIT EINEM BASISCHEN MEDIUM
COMPOSITION PHARMACEUTIQUE STABILISEE A L'AIDE D'UN AGENT DE BASE

(30) Priority: 22.12.1995 JP 35465495
(43) Date of publication of application: 07.01.1998
(73) Proprietor: KOWA COMPANY LTD., Nagoya-shi Aichi-ken, 460-8625 (JP); NISSAN CHEMICAL INDUSTRIES, LIMITED, Chiyoda-ku Tokyo 101 (JP)
(72) Inventor: MURAMATSU, Toyojiro, Shizuoka 422 (JP); MASHITA, Katsumi, Shizuoka 417 (JP); SHINODA, Yasuo, Shizuoka 420 (JP); SASSA, Hironori, Shizuoka 420 (JP); KAWASHIMA, Hiroyuki, Shizuoka 417 (JP); TANIZAWA, Yoshio, Okayama 701-13 (JP); TAKEUCHI, Hideatsu, Shizuoka 417 (JP)
(74) Representative: Silverman, Warren
(86) International application number: JP9603722
(87) International publication number: WO97023200

(56) References cited:
- EP-A- 0 304 063
- EP-A- 0 336 298
- EP-A- 0 446 961
- EP-A- 0 520 406
- US-A- 5 356 896

## Description

### TECHNICAL FIELD

The present invention relates to a pharmaceutical composition with high stability and, more precisely, to a pharmaceutical composition comprising an HMG-CoA reductase inhibitor of which the stability varies depending on its pH, especially (E)-3,5-dihydroxy-7-[4'-4"-fluorophenyl-2'-cyclopro pyl-quinolin-3'-yl]-6-heptenoic acid, or its salt or ester.

### BACKGROUND ART

It is known that 7-substituted-3,5-dihydroxy-6-heptenoic acids of a general formula: 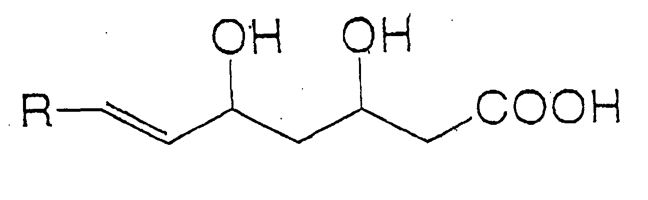 wherein R represents an organic group, have HMG-CoA reductase-inhibiting activity, and are useful as medicines for hyperlipemia and also as medicines for atherosclerosis (see U.S. Patent 4,739,073, U.S. Patent 5,001,255, U.S. Patent 4,751,235, U.S. Patent 4,804,679, Japanese Patent Application Laid-Open No. 1-279866).

However, these 7-substituted-3,5-dihydroxy-6-heptenoic acids are unstable at low pH, and require some particular means for formulating them into preparations. A means of formulating them along with an alkaline medium, such as calcium carbonate or sodium carbonate, into preparations with pH of 8 or higher (see Japanese Patent Application Laid-Open No. 5-246844), and a means of formulating them along with a basic agent, such as magnesium oxide or sodium hydroxide, into preparations with pH of 9 or higher (see Japanese Patent Application Laid-Open No. 2-6406) have been proposed.

(E)-3,5-dihydroxy-7-[4'-4"-fluorophenyl-2'-cyclopropy 1-quinolin-3'-yl]-6-heptenoic acid (hereinafter this may be referred to as NK-104) to be represented by a structural formula: 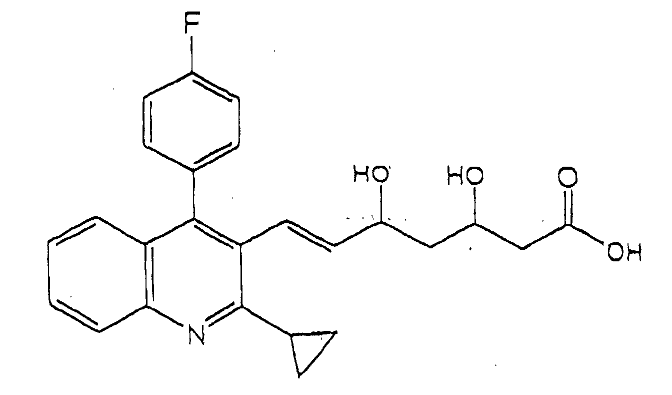 or its salt or ester is one of HMG-CoA reductase inhibitors that are represented by the above-mentioned general formula, and is known to be useful as a medicine for hyperlipemia and also as a medicine for atherosclerosis (see Japanese Patent Application Laid-Open No. 1-279866). NK-104 is also unstable at low pH, and many difficulties have been encountered in formulating it into preparations.

It has been reported that these HMG-CoA reductase inhibitors are formulated into preparations with pH 8 or higher, desirably pH 9 or higher, but unexpectedly, it has been found that NK-104 and its salts and esters are still unstable even within a high pH range.

Therefore, preparations comprising NK-104 or its salt or ester, if formulated in conventional manners, have low time-dependent stability, and are problematic in that their outward appearance changes with the lapse of time. Given the situation, the development of stable preparations comprising it is desired.

### SUMMARY OF THE INVENTION

We, the present inventors have variously studied in order to obtain stable pharmaceutical compositions comprising NK-104 and, as a result, have found unexpectedly that NK-104 is stable within a relatively low pH range. On the basis of this finding, we have completed the present invention.

In particular, we have found that, if a basic substance is added to a pharmaceutical composition comprising NK-104 or a salt or ester thereof in such a manner that an aqueous solution or dispersion of the composition has a pH of from 7 to 7.8, the composition is stable.

The subject of the present invention, is therefore a pharmaceutical composition comprising NK-104, or a salt or ester thereof to which is added a basic substance to make an aqueous solution or dispersion thereof have a pH of from 7.0 to 7.8, preferably of from 7.1 to 7.8, when it is dispersed in 1 to 10ml of water.

The active ingredient of the composition of the present invention is NX-104 to be represented by the above-mentioned structural formula. The configuration in this substance, NK-104 is not specifically defined herein. In addition, NK-104 may be in any form of its salts and esters. The salts include, for example, sodium salt, potassium salt and calcium salt. Preferred is calcium salt of NX-104.

### DETAILED DESCRIPTION OF THE INVENTION

The pH as referred to herein indicates the pH value is to be determined in such a manner that a unit dose of a solid preparation comprising NK-104 or its salt or ester is sampled and dissolved or dispersed in from 1 to 10 ml of pure water, and the pH of the resulting aqueous solution or dispersion is measured.

A basic substance may be added to the pharmaceutical composition comprising NH-104 to control the pH of the composition, which may be any of antacids and pH regulators including, for example, antacids such as magnesium aluminometasilicate, magnesium aluminosilicate, magnesium aluminate, dry aluminium hydroxide, synthetic hydrotalcite, synthetic aluminium silicate, magnesium carbonate, precipitated calcium carbonate, magnesium oxide, aluminium hydroxide, and sodium hydrogencarbonate; and pH regulators such as L-arginine, sodium phosphate, disodium hydrogenphosphate, sodium dihydrogenphosphate, potassium phosphate, dipotassium hydrogenphosphate, potassium dihydrogenphosphate, disodium citrate, sodium succinate, ammonium chloride, and sodium benzoate. Of these, preferred are magnesium metasilicate aluminate, L-arginine, and dipotassium hydrogenphosphate.

The pharmaceutical composition of the present invention can be formulated into,various forms of preparations, but preferred are peroral solid preparations. For example, the composition may be formulated into tablets, granules, powders, troches, capsules, chewables, film-coated preparations of these, and even sugar-coated preparations thereof.

Where the pharmaceutical composition of the present invention is formulated into such peroral solid preparations, any of vehicles (excipients), binders, disintegrators and lubricants can be added thereto, if desired. The preparations may be formulated from the composition along with any of these, in any ordinary manner.

The vehicles (excipients) include, for example, lactose, corn starch, denatured corn starch, mannitol, sorbitol, wood cellulose, fine crystalline cellulose and calcium carbonate, which can be used either singly or as combined.

The binders include, for example, hydroxypropyl cellulose, hydroxypropylmethyl cellulose, polyvinyl pyrrolidone, polyvinyl alcohol, and partial saponificates of these, which can be used either singly or as combined. Especially preferred is hydroxypropylmethyl cellulose.

The disintegrators include, for example, hydroxypropyl cellulose with a low degree of substitution, carmellose, sodium carboxystarch, calcium carmellose, corn starch, partially-alphatized starch, sodium closcarmellose and clospovidone, which can be used either singly or as combined. Especially preferred is Low substituted hydroxypropyl cellulose.

The lubricants includes, for example, magnesium stearate, stearic acid, palmitic acid, calcium stearate and talc, which can be used either singly or as combined.

The amounts of the ingredients constituting the composition of the present invention are not specifically defined. For example, the amount of NK-104 or its salt or ester may be from 0.01 to 40 % by weight, preferably from 0.05 to 10 % by weight, more preferably from 0.5 to 5 % by weight; and the basic substance may be added to the composition in such an amount that is necessary for making the aqueous solution or dispersion of the composition have pH of from 7 to 7.8. Where the composition is formulated into peroral solid preparations, it is desirable that the vehicle is added thereto in an amount of from 30 to 95 % by weight, the binder in an amount of from 1 to 20 % by weight, the disintegrator in an amount of from 1 to 30 % by weight, and the lubricant in an amount of from 0.5 to 10 % by weight.

If further desired, any additional components, such as sweeteners, flavorings and colorants may also be added to the composition of the present invention.

The necessary amount of the basic substance to be added to the composition of the invention in order to make the aqueous solution or dispersion of the composition have pH of from 7 to 7.8 may be from about 1 to 6.5 % by weight or so, if magnesium metasilicate aluminate is used singly, or from about 0.1 to 1.7 % by weight or so, if dipotassium hydrogenphosphate is used singly, of from about 0.01 to 0.1 % by weight or so, if L-arginine is used singly, or from about 0.1 to 2 % by weight or so, if sodium hydrogencarbonate is used singly. As mentioned above, it is preferable that the basic substance is used singly. However, two or more such basic substances can be used in combination.

The composition of the present invention can be coated to give film-coated tablets or sugar-coated tablets. As the coating base, for example, usable are celluloses such as hydroxypropyl cellulose, hydroxypropylmethyl cellulose; and also aminoalkyl methacrylate copolymer E, white sugar, and pullulan. As the plasticizer for the base, for example, usable are macrogol 6000, triethyl citrate, and triacetylpropylene glycol.

The pharmaceutical composition of the present invention can be produced according to any ordinary methods employable in producing peroral solid preparations. If stirring granulation is employed, this may be conducted as follows. First, NK-104, a basic substance, a vehicle, a binder and a disintegrator are mixed. Next, water is added to the resulting mixture, then granulated with stirring, dried and dressed to give dry granules. Further, the granules are mixed with a lubricant, and pelletized with a pelletizer into pellets. Also employable is fluidized bed granulation, which may be conducted as follows. First, NK-104, a basic substance, a vehicle and a disintegrator are mixed. Then, an aqueous solution of a binder is sprayed over the resulting mixture, using a fluidized bed granulator, to prepare granules. These granules are mixed with a lubricant, and then pelletized with a pelletizer into pellets.

Using ordinary coating devices, the pellets as produced according to the above-mentioned methods can be coated with a solution or suspension comprising a coating base and optionally a plasticizer and a colorant to give film-coated tablets or sugar-coated tablets.

### BEST MODES OF CARRYING OUT THE INVENTION

Examples of the pharmaceutical composition of the present invention are mentioned below. In the following examples, the low substituted hudroxypropyl cellulose was commercially available as sold for a medicine additive and contains from 5-16% of OC₃H₆OH group. Hydroxypropylmethyl cellulose 2910 contains 28-30% OCH₃ and 7-12% OC₃H₆OH. Both low substituted hydroxypropyl cellulose and hydroxypropylmethyl cellulose 2910 as used in the examples are described in The Pharmacopoeia of Japan, 12th edition.

### Example 1:

Herein produced were tablets each having the composition mentioned below.

| | |
|---|---|
| Calcium Salt of NX-104 | 1.0 mg |
| Lactose | 101.4 |
| Low Substituted Hydroxypropyl Celulose | 12.0 |
| Hydroxypropylmethyl Cellulose 2910 | 2.0 |
| Magnesium Metasilicate Aluminate | 2.4 |
| Magnesium Stearate | 1.2 |
| Total (one tablet) | 120.0 |

The components of the above-mentioned composition, except magnesium stearate, were mixed to prepare a homogeneous powdery mixture, to which was added a suitable amount of pure water. The resulting mixture was granulated with stirring, and palletized to give pellets. Magnesium stearate was added to and mixed with these pellets, which were then tabletted into NK-104-containing tablets

### Example 2:

In the same manner as in Example 1, herein produced were tablets each having the composition mentioned below.

| | |
|---|---|
| Calcium Salt of NK-104 | 1.0 mg |
| Lactose | 102.8 |
| Low Substituted Hydroxypropyl Cellulose | 12.0 |
| Hydroxypropylmethyl Cellulose 2910 | 2.0 |
| Dipotassium Hydrogenphosphate | 1.0 |
| Magnesium Stearate | 1.2 |
| Total (one tablet) | 120.0 |

### Example 3:

In the same manner as in Example 1, herein produced were tablets each having the composition mentioned below.

| | |
|---|---|
| Calcium Salt of NK-104 | 1.0 mg |
| Lactose | 103.7 |
| Low Substituted Hydroxypropyl Cellulose | 12.0 |
| Hydroxypropylmethyl Cellulose 2910 | 2.0 |
| L-arginine | 0.1 |
| Magnesium Stearate | 1.2 |
| Total (one tablet) | 120.0 |

### Example 4:

In the same manner as in Example 1, herein produced were tablets each having the composition mentioned below.

| | |
|---|---|
| Calcium Salt of NK-104 | 1.0 mg |
| Lactose | 103.2 |
| Law Substituted Hydroxypropyl Cellulose | 12.0 |
| Hydroxypropylmethyl Cellulose 2910 | 2.0 |
| Magnesium Metasilicate Aluminate | 0.6 |
| Magnesium Stearate | 1.2 |
| Total (one tablet) | 120.0 |

### Test 1:

The pH of a 5 % suspension of tablets produced in any of Examples 1 to 4 (the suspension was prepared by suspending one tablet in 2.4 ml of pure water) was measured.

After having been stored at 60°C for 2 weeks, the percentage retention of calcium salt of NK-104 in the tablets was measured according to HPLC. After having been stored at 60°C for 3 days, the change in the outward appearance of the tablets was observed. The test results are shown in Table I.

**Table 1**

| | Example 1 | Example 2 | Example 3 | Example 4 |
|---|---|---|---|---|
| pH of 5% Suspension | 7.8 | 7.7 | 7.6 | 7.1 |
| Percentage Retention of Ca NK-104 | 97 % | 97 % | 93 % | 92 % |
| Change In Outward Appearance | No change | No change | No change | No change |

### Control Examples 1 to 3:

In the same manner as in Example 1, herein produced were control tablets each having the composition mentioned below. These tablets were tested in the same manner as in Test 1, to determine the pH of the 5 % suspension of each tablet, the percentage retention of Ca NK-104, and the change in the outward appearance of the tablets. The test results are shown in Table 2.

**Table 2**

| | Control Example 1 | Control Example 2 | Control Example 3 |
|---|---|---|---|
| Ca NK-104 | 1.0 mg | 1.0 mg | 1.0 mg |
| Lactose | 103.8 | 98.6 | 98.8 |
| Hydroxypropyl Cellulose with Low Degree of Substitution | 12.0 | 12.0 | 12.0 |
| Hydroxypropylmethyl Cellulose 2910 | 2.0 | 2.0 | 2.0 |
| Sodium Ascorbate | | 5.0 | |
| Ascorbic Acid | | | 5.0 |
| Magneslum Stearate | 1.2 | | |
| Total (one tablet) | 120.0 | 120.0 | 120,0 |
| pH of 5 % Suspension | 6.6 | 6.3 | 3.3 |
| Percentage Retention of Ca NK-104, after stored at 60°C for 2 weeks | 88 % | 77 % | 38 % |
| Change In Outward Appearance, after stored at 60°C for 3 days | No change | No change | No change |

As in Tables 1 and 2 showing the test results, it is obvious that the percentage retention of Ca NK-104 in the 5 % suspension of the composition having pH of 7 or higher is high, after having been stored at 60°C for 2 weeks, while the same in the 5 % suspension thereof having pH of lower than 7 becomes lower with the decrease in the pH value thereof.

### Example 5, and Control Example 4:

In the same manner as in Example 1, herein produced were tablets each having the composition mentioned below. These tablets were tested in the same manner as in Test 1, to determine the pH of the 5 % suspension of each tablet, and the change in the outward appearance of the tablets. The test results are shown in Table 3.

**Table 3**

| | Example 5 | Control Example 4 |
|---|---|---|
| Ca NK·104 | 1.0 mg | 1.0 mg |
| Lactose | 101.4 | 93.9 |
| Hydroxypropyl Cellulose with Low Degree of Substitution | 12.0 | 12.0 |
| Hydroxypropylmethyl Cellulose 2910 | 2.0 | 2.0 |
| Magnesium Metasilicate Aluminate | 2.4 | 9.9 |
| Magnesium Stearate | 1.2 | 1.2 |
| Total (one tablet) | 120.0 | 120.0 |
| pH of 5 % Suspension | 7.8 | 8.3 |
| Change in Outward Appearance, after stored at 60°C for 3 days | No change | Changed to pale yellowish brown |

### Example 6, and Control Example 5:

In the same manner as in Example 1, herein produced were tablets each having the composition mentioned below. These tablets were tested in the same manner as in Test 1, to determine the pH of the 5 % suspension of each tablet, and the change in the outward appearance of the tablets. The test results are shown in Table 4.

**Table 4**

| | Example 6 | Control Example 5 |
|---|---|---|
| Ca NK-104 | 1.0 mg | 1.0 mg |
| Lactose | 103.7 | 93.9 |
| Hydroxypropyl Cellulose with Low Degree of Substitution | 12.0 | 12.0 |
| Hydroxypropylmethyl Cellulose 2910 | 2.0 | 2.0 |
| L-arginine | 0.1 | 9.9 |
| Magnesium Stearate | 1.2 | 1.2 |
| Total (one tablet) | 120.0 | 120.0 |
| pH of 5 % Suspension | 7.5 | 9.8 |
| Change In Outward Appearance, after stored at 60°C for 3 days | No change | Changed to pale yellowish green |

### Example 7, and Control Example 6:

In the same manner as in Example 1, herein produced were tablets each having the composition mentioned below. These tablets were tested in the same manner as in Test 1, to determine the pH of the 5 % suspension of each tablet, and the change in the outward appearance of the tablets. The test results are shown in Table 5.

**Table 5**

| | Example 7 | Control Example 6 |
|---|---|---|
| Ca NK-104 | 1,0 mg | 1.0 mg |
| Lactose | 101,8 | 93,9 |
| Hydroxypropyl Cellulose with Low Degree of Substitution | 12.0 | 12.0 |
| Hydroxypropylmethyl Cellulose 2910 | 2.0 | 2.0 |
| Sodium Hydrogencarbonate | 2.0 | 9.9 |
| Magnesium Stearate | 1.2 | 1.2 |
| Total (one tablet) | 120.0 | 120.0 |
| pH of 5 % Suspension | 7.8 | 8.8 |
| Change In Outward Appearance, after stored at 60°C for 3 days | No change | Changed to dark navy blue |

### Example 8, and Control Example 7:

In the same manner as in Example 1, herein produced were tablets each having the composition mentioned below. These tablets were tested in the same manner as in Test 1, to determine the pH of the 5 % suspension of each tablet, and the change in the outward appearance of the tablets. The test results are shown in Table 6.

**Table 6**

| | Example 8 | Control Example 7 |
|---|---|---|
| Ca NK-104 | 1.0 mg | 1.0 mg |
| Lactose | 102.8 | 93.9 |
| Hydroxypropyl Cellulose with Low Degree of Substitution | 12.0 | 12.0 |
| Hydroxypropylmethyl Cellulose 2910 | 2.0 | 2.0 |
| Dipotassium Hydrogenphosphate | 1.0 | 9.9 |
| Magnesium Stearate | 1.2 | 1.2 |
| Total (one tablet) | 120.0 | 120.0 |
| pH of 5 % Suspension | 7.7 | 8.4 |
| Change in Outward Appearance, after stored at 60°C for 3 days | No change | Changed to orange |

As is obvious from the test results in Tables 3 to 6, no change in the outward appearance of the tablets was found when the 5 % suspensions of the tablets had pH of below 8, even after having been stored at 60°C for 3 days, but the outward appearance of the tablets changed when the 5 % suspensions of the tablets had pH of higher than 8.

### INDUSTRIAL APPLICABILITY OF THE INVENTION

The pharmaceutical composition of the present invention has good time-dependent stability, with having no change in the outward appearance thereof even after having been stored long. Therefore, the composition is good in medical use, especially in the form of peroral solid preparations.

The pharmaceutical composition of the present invention that contains NK-104 or salt or ester thereof are especially useful for treating a patient, particularly a human, that is suffering from or susceptible to hyperlipemia or atherosclerosis by administering the pharmaceutical composition to such patient.

Particularly preferred unit dosage have been described in the examples above. It will be appreciated the specifically preferred dosage amounts of a pharmaceutical composition of the invention used in a given therapy will vary according to various known factors such as the particular compositions formulated, the specific compound utilized, the mode of application, the particular site of administration, etc. Optimal administration rates for a given protocol of administration can be readily ascertained by those skilled in the art using conventional dosage determination tests conducted with regard to the foregoing guidlines.

## Claims

1. A pharmaceutical composition comprising (E)-3,5-dihydroxy-7-[4'-4"-fluorophenyl-2'-cyclopropyl-quinolin-3'-yl]-6-heptenoic acid, or its salt or ester, to which is added a basic substance to make an aqueous solution or dispersion of the composition have a pH of from 7 to 7.8 when it is dispersed in 1 to 10ml of water.

2. The pharmaceutical composition as claimed in claim 1, wherein the basic substance is added to make the aqueous solution or dispersion of the composition have a pH of from 7.1 to 7.8.

3. The pharmaceutical composition as claimed in claim 1 or 2, wherein the salt of (E)-3,5-dihydroxy-7-[4'-4"-fluoro-phenyl-2'-cyclopropyl-quinolin-3'-yl)-6-heptenoic acid is the calcium salt of the acid.

4. The pharmaceutical composition as claimed in claim 1, 2 or 3, wherein the basic substance is one or more selected from antacids and pH regulators.

5. The pharmaceutical composition as claimed in claim 4, wherein the antacid is magnesium aluminometasilicate.

6. The pharmaceutical composition as claimed in claim 4 or 5, wherein the pH regulator is L-arginine or dipotassium hydrogenphosphate.

7. The pharmaceutical composition as claimed in any one of claims 1 to 6, which further contains at least one of vehicles, disintegrators, binders and lubricants.

8. The pharmaceutical composition as claimed in any one of claims 1 to 7, which is a peroral solid preparation.

9. The pharmaceutical composition as claimed in claim 7 or 8, wherein the vehicle is lactose.

10. The pharmaceutical composition as claimed in any one of claims 7 to 9, wherein the disintegrator is hydroxypropyl cellulose with a low degree of substitution.

11. The pharmaceutical composition as claimed in any one of claims 7 to 10, wherein the binder is hydroxypropylmethyl cellulose.

## Patentansprüche

1. Pharmazeutische Zusammensetzung, umfassend (E)-3,5-Dihydroxy-7-[4'-4'-fluorphenyl-2'-cyclopropylchinolin-3'-yl]-6-heptensäure oder deren Salze oder Ester, zu welcher zugesetzt ist ein basischer Stoff, so dass man eine wässrige Lösung oder Dispersion der Zusammensetzung erhält, wobei der pH zwischen 7 und 7,8 liegt, wird sie in 1 bis 10 ml Wasser dispergiert.

2. Pharmazeutische Zusammensetzung nach Anspruch 1, wobei die basische Substanz so zugesetzt wird, dass man eine wässrige Lösung oder Dispersion der Zusammensetzung mit einem pH von 7,1 bis 7,8 erhält.

3. Pharmazeutische Zusammensetzung nach Anspruch 1 oder 2, wobei das Salz der (E)-3,S-Dihydroxy-7-[4'-4''fluorphenyl-2'-cyclopropyl-chinolin-3'-yl]-6-heptensäure das Calciumsalz der Säure ist.

4. Pharmazeutische Zusammensetzung nach Anspruch 1, 2 oder 3, wobei die basische Substanz ausgewählt ist aus ein oder mehreren Antacida sowie pH-Regulatoren.

5. Pharmazeutische Zusammensetzung nach Anspruch 4, wobei das Antacid Magnesiumaluminiummetasilicat ist.

6. Pharmazeutische Zusammensetzung nach Anspruch 4 oder 5, wobei der pH-Regulator L-Arginin oder Dikaliumhydrogenphosphat ist.

7. Pharmazeutische Zusammensetzung nach irgendeinem der Ansprüche 1 bis 6, die zudem mindestens einen Stoff enthält aus Trägersubstanzen, Zerfallsmitteln, Bindemitteln oder Gleitmitteln.

8. Pharmazeutische Zusammensetzung nach irgendeinem der Ansprüche 1 bis 7, welche eine perorale Feststoffzubereitung ist.

9. Pharmazeutische Zusammensetzung nach Anspruch 7 oder 8, wobei der Träger Lactose ist.

10. Pharmazeutische Zusammensetzung nach irgendeinem der Ansprüche 7 bis 9, wobei das Zerfallsmittel Hydroxypropylcellulose mit niedrigem Substitutionsgrad ist.

11. Pharmazeutische Zusammensetzung nach irgendeinem der Ansprüche 7 bis 10, wobei das Bindemittel Hydroxypropylmethylcellulose ist.

## Revendications

1. Composition pharmaceutique comportant de l'acide (E)-3,5-dihydroxy-7-[4'-4"-fluorophényle-2'-cyclopropyle-quinoline-3'-yle]-6-hep tènoïque, ou son sel ou ester, à laquelle est ajoutée une substance basique pour amener une solution ou dispersion aqueuse de la composition à avoir un pH compris entre 7 et 7,8 lorsqu'elle est dispersée dans 1 à 10 ml d'eau.

2. Composition pharmaceutique selon la revendication 1, dans laquelle la substance basique est ajoutée pour amener la solution ou dispersion aqueuse de la composition à avoir un pH compris entre 7,1 et 7,8.

3. Composition pharmaceutique selon la revendication 1 ou 2, dans laquelle le sel de l'acide (E)-3,5-dihydroxy-7-[4'-4"-fluorophényle-2'-cyclopropyle-quinoline-3'-yle]-6-heptènoïque est le sel de calcium de l'acide.

4. Composition pharmaceutique selon la revendication 1, 2 ou 3, dans laquelle la substance basique est un ou plusieurs composés sélectionnés parmi des antiacides et des régulateurs de pH.

5. Composition pharmaceutique selon la revendication 4, dans laquelle l'antiacide est un alumino-métasilicate de magnésium.

6. Composition pharmaceutique selon la revendication 4 ou 5, dans laquelle le régulateur de pH est une L-arginine ou un hydrogènephosphate de dipotassium.

7. Composition pharmaceutique selon l'une quelconque des revendications 1 à 6, qui comporte de plus au moins un élément parmi des véhicules, des désintégrateurs, des liants et des lubrifiants.

8. Composition pharmaceutique selon l'une quelconque des revendications 1 à 7, qui est une préparation solide perorale.

9. Composition pharmaceutique selon la revendication 7 ou 8, dans laquelle le véhicule est du lactose.

10. Composition pharmaceutique selon l'une quelconque des revendications 7 à 9, dans laquelle le désintégrateur est une hydroxypropylcellulose ayant un faible degré de substitution.

11. Composition pharmaceutique selon l'une quelconque des revendications 7 à 10, dans laquelle le liant est une hydroxypropylméthylcellulose.
